(19)

Europäisches Patentamt

European Patent Office

Office européen des brevets

(11) **EP 2 749 655 B2**

(12) **NEW EUROPEAN PATENT SPECIFICATION**

After opposition procedure

(45) Date of publication and mention
of the opposition decision:
**13.04.2022  Bulletin 2022/15**

(45) Mention of the grant of the patent:
**03.07.2019  Bulletin 2019/27**

(21) Application number: **12825673.2**

(22) Date of filing: **24.08.2012**

(51) International Patent Classification (IPC):
*C12Q 1/6827* (2018.01)      *G16B 20/00* (2019.01)
*G16B 30/00* (2019.01)      *C12Q 1/6869* (2018.01)

(52) Cooperative Patent Classification (CPC):
**G16B 30/00; C12Q 1/6827; C12Q 1/6869;
G16B 20/00; G16B 20/20; G16B 30/10**

(86) International application number:
**PCT/CN2012/080578**

(87) International publication number:
**WO 2013/026411 (28.02.2013 Gazette 2013/09)**

(54) **SINGLE CELL CLASSIFICATION METHOD, GENE SCREENING METHOD AND DEVICE THEREOF**

EINZELZELLENKLASSIFIKATIONSVERFAHREN, GEN SCREENING-VERFAHREN UND
VORRICHTUNG DAFÜR

PROCÉDÉ DE CLASSIFICATION DE CELLULE INDIVIDUELLE, PROCÉDÉ DE CRIBLAGE DE GÈNE
ET DISPOSITIF POUR CELUI-CI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.08.2011  CN 201110245356**

(43) Date of publication of application:
**02.07.2014  Bulletin 2014/27**

(73) Proprietors:
• **BGI Shenzhen Co., Limited
Shenzhen, Guangdong 518083 (CN)**
• **BGI Shenzhen
Shenzhen, Guangdong 518083 (CN)**

(72) Inventors:
• **XU, Xun
Shenzhen
Guangdong 518083 (CN)**
• **BAO, Li
Shenzhen
Guangdong 518083 (CN)**
• **HE, Weiming
Shenzhen
Guangdong 518083 (CN)**
• **HOU, Yong
Shenzhen
Guangdong 518083 (CN)**
• **TAO, Ye
Shenzhen
Guangdong 518083 (CN)**

(74) Representative: **HGF
HGF Limited
1 City Walk
Leeds LS11 9DX (GB)**

(56) References cited:
**CN-A- 101 914 628      CN-A- 101 956 006
CN-A- 102 061 526**

• **NICHOLAS NAVIN ET AL: "Tumour evolution
inferred by single-cell sequencing", NATURE,
vol. 472, no. 7341, 13 March 2011 (2011-03-13),
pages 90-94, XP55042936, ISSN: 0028-0836, DOI:
10.1038/nature09807**
• **NATHAN A. BAIRD ET AL: "Rapid SNP Discovery
and Genetic Mapping Using Sequenced RAD
Markers", PLOS ONE, vol. 3, no. 10, 1 January
2008 (2008-01-01), page e3376, XP055005983,
ISSN: 1932-6203, DOI:
10.1371/journal.pone.0003376**
• Nicholas Navin et al: "Tumour evolution inferred
by single-cell sequencing", Nature, Macmillan
Journals Ltd., etc.|, vol. 472, no. 7341, 7 April 2011
(2011-04-07), pages 90-94, ISSN: 00280836, DOI:
10.1038/nature09807
• **Rasmus Nielsen, Joshua S. Paul, Anders
Albrechtsen, Yun S. Song: "Genotype and SNP
calling from next-generation sequencing data",
Nature Reviews Genetics, Nature Pub. Group, vol.
12, no. 6, 1 June 2011 (2011-06-01), pages 443-451,
ISSN: 14710056, DOI: 10.1038/nrg2986**

EP 2 749 655 B2

- **R. Li, Y. Li, X. Fang, H. Yang, J. Wang, K. Kristiansen: "SNP detection for massively parallel whole-genome resequencing", Genome Research,, vol. 19, no. 6, 1 June 2009 (2009-06-01), pages 1124-1132, ISSN: 10889051, DOI: 10.1101/gr.088013.108**
- **R. Li, Et Al: "SOAP2: an improved ultrafast tool for short read alignment", Bioinformatics, vol. 25, no. 15, 1 January 2009 (2009-01-01), pages 1966-1967,**

## Description

### Technical Field

The present invention relates to bioinformatics, and in particular, relates to a single cell classification method and device used for said method.

### Background

Significant differences exist in gene expression, copy number variation, epigenetics and the like among different individuals, among different tissues of an individual, and even different sites of the same tissue. The heterogeneity also exists among cells, even for a cell group cultured *in vitro* with exactly the same genetic background. Since any state changes are heritable for stem cells or precursor cells, the cell heterogeneity is particularly evident. In order to better study cell biology, and reveal the rules of the cell heterogeneity, it is greatly needed to develop a technical method applied to single cell studies, and thus some scholars have proposed the concept of "single cell analysis (SCA)" for elaboration from the angle of "omics". Single cell classification and screening provide an important foundation for the single cell analysis.

The single cell classification can be effectively applied in studies on differentiation processes of various kinds of stem cells, e.g., in studies on directional differentiation of tumor stem cells and embryonic stem cells, and hemopoietic stem cells, the stem cells of different differentiation stages need to be screened and various kinds of stem cells need to be detected. In drug resistance studies, the cells at different periods of drug-administration need to be precisely classified, so as to further analyze the drug resistance and drug resistance genes of the cell subgroups, e.g., studies can be performed on the relationship between the multidrug resistance and multidrug resistance genes of a cancer patient and drug abuse, drug tolerance and drug dependence. Likewise, in the screening for a drug target gene, because the interaction between a drug and cells, in particular, sensitive cells, will cause a series of changes in the external morphology and the internal normal metabolic processes of the cells, and thus screening to obtain sensitive cells is a key first step, which provides an important foundation for precisely locating the drug target gene at the late stage. The single cell classification and screening are applied to the establishment of a pharmacodynamic screening model, provide a theoretical basis for the drug design, the target selection and the determination of a dosage regimen, and furthermore make the drug screening have a higher specificity.

Currently, the commonly used single cell screening methods are mostly physical and mechanical, chemical or biological methods, e.g., a flow cytometer, a magnetic cell sorter and other methods. On one hand, these techniques adopt surfactants, fluorescent dyes and antigens and antibodies, are of high cytotoxicity, and can only sort a suspension of specifically labeled or non-specifically labeled single cells, with cumbersome sample preparation processes at the early stage; and currently, there is relatively much disputation on the specificity of numerous fluorescent probes and monoclonal antibodies (including CD molecules on cell surfaces), and many cell subgroups have no corresponding specific marker/specific antigen. On the other hand, these techniques adopt biological, immunological and chemical methods to perform statistical analysis by phenotype determination (including the cell size, the cell particle size, the cell surface area, the nucleus-cytoplasm ratio and the like), and the sensitivity of subgroup classification, screening and detection is low, thus lacking an effective assessment of accuracy.

### SUMMARY OF THE INVENTION

The present invention is aimed at solving at least one of the technical problems existing in the prior art.

Accordingly, the present invention provides a method of classifying single cells as set forth in claim 1 or claim 2. Further, the invention provides a device for classifying single cells by genotype as set forth in any of claims 3 to 5.

In the present invention, unless otherwise stated, the scientific and technical terms used herein have the meanings commonly understood by a person skilled in the art. At the same time, for the present invention to be understood better, the definitions and explanations of relevant terms are provided below.

The term "file on the possibility of genotypes" means a collection of numerical values of posteriori probabilities of possible genotypes calculated for a sample in a target region by using SNP detection software, setting priori probability parameters and using the Bayesian formula; and when the used SNP detection software is SOAPsnp, the generated "file on the possibility of genotypes" is a CNS file.

As used herein, a "genotype file" means a collection of genotypes of group SNPs at corresponding sites of various cells obtained by extracting the sites corresponding to the genotype of each cell according to the position information in an SNP dataset of a reference genome, after selecting a genotype with a maximum probability in the above-mentioned "file on the possibility of genotypes" as a consistent genotype of each cell.

Described herein is a single cell classification method. comprising: sequencing the whole genomes of a plurality of single cell samples from the same group, respectively, so as to obtain reads from each single cell sample; aligning the reads from each single cell sample to the sequence of a reference genome, respectively, and performing data filtering on said reads; on the basis of the filtered reads, determining a consistent genotype of each single cell sample, in which consistent genotypes of all the single cell samples constitute an SNP dataset of said group; aimed at said each single cell, on the basis of the SNP dataset of said group, determining a corresponding genotype for each cell at a site corresponding to a position in an SNP dataset of the reference genome; and selecting an SNP site associated with cell mutation, and on the basis of the genotypes of said single cells at the site, classifying said single cells. Thus, as described herein the next-generation sequencing (NGS) technology can be adopted through the bioinformatics methods to analyze and study single cell genomes, and to collect cell subgroups (or microparticles) to perform subsequent in-depth studies. On one hand, the operation of labeling cells is avoided, which effectively solves the problem in traditional single cell classification methods that certain cell subgroups have no corresponding specific marker and cannot be classified; on the other hand, the genetic variation information of single cell genomes is analyzed comprehensively and completely, and a plurality of control samples can be set, which greatly increases the accuracy of cell subgroup classification.

Said sequencing may be performed using a second-generation or third-generation sequencing platform, and the criteria of said data filtering are: when a plurality of duplicated paired-end reads are present, and the sequences of the plurality of pairs of paired-end reads are fully consistent, randomly selecting one pair of reads, and removing the other duplicated paired-end reads in said plurality of pairs of paired-end reads; and/or removing reads which are not uniquely aligned onto the sequence of said reference genome.

On the basis of the filtered reads, determining a consistent genotype of each single cell further may include: on the basis of said filtered reads, determining a possibility of a genotype of each single cell sample in a target region; on the basis of possibilities of genotypes of all the single cell samples in the target region, determining a pseudo-genome containing each site of all the samples; and selecting a genotype with a maximum probability from said pseudo-genome as the consistent genotype of each single cell sample.

Selecting an SNP site associated with cell mutation further removes at least one of the following items from the SNP dataset of said group: non inter-group SNP sites, sites of loss of heterozygosity, and published SNP sites.

The whole genome of at least one of said plurality of single cell samples is subjected to the whole genome amplification treatment before being sequenced, in which removing sites of loss of heterozygosity may further include removing sites that meet the following conditions in samples that have not undergone whole genome amplification, the sequencing results being heterozygous sites; and in samples that have undergone whole genome amplification, at the same site, the number of samples with loss of heterozygous sites and data being greater than or equal to the number of the samples that have undergone whole genome amplification minus 3.

As described herein, the method is aimed at said each single cell, on the basis of the SNP dataset of said group, determining a corresponding genotype for each cell at a site corresponding to a position in an SNP dataset of the reference genome, further includes screening said SNP dataset according to the following criteria: the quality value of the consistent genotype of each site being not less than 20, and the p value for the rank test being not less than 1%; and for SNPs of heterozygous variation: the major allele's sequencing quality value being not less than 20, and the sequencing depth being not less than 6, the minor allele's sequencing quality value being not less than 20, the sequencing depth being not less than 2, and the ratio of sequencing depths of two genotypes being within a range of 0.2-5.

After classifying cells, the following steps may also be included: extracting the information of each cell sample, and excluding contentious cells.

After classifying said single cells, the following steps may be further included: determining the classified groups on the basis of the classification result, and calculating a statistic of all SNP sites of each gene in each class of groups, optionally performing a difference test on the obtained statistic to obtain a test value; and selecting a gene with the highest statistic or test value as a gene associated with cell mutation.

Also described herein, is a single cell classification device compriseing: a data filtering module, said data filtering module being suitable for aligning reads from each single cell sample to the sequence of a reference genome, respectively, and performing data filtering on said reads, in which the reads of said each single cell sample are obtained by sequencing the whole genomes of a plurality of single cell samples, respectively; a genotype determination module, said genotype determination module being suitable for determining a consistent genotype of each single cell sample on the basis of the filtered reads, in which consistent genotypes of all the single cell samples constitute an SNP dataset of said group; a genotype file extraction module, said genotype file extraction module being suitable for aimed at said each single cell, on the basis of the SNP dataset of said group, determining a corresponding genotype for each cell at a site corresponding to a position in an SNP dataset of the reference genome; and a classification module, said classification module being suitable for classifying said single cells on the basis of a pre-selected SNP site associated with cell mutation, and on the basis of the genotypes of said single cells at the site. Using the device can effectively implement the aforementioned single cell classification method. Next-generation sequencing (NGS) technology may be adopted through the bioinformatics methods to analyze and study single cell genomes, and to collect cell subgroups (or microparticles) to perform subsequent in-depth studies. On one hand, the operation of labeling cells is avoided, which effectively solves the problem in traditional single cell classification methods that certain cell subgroups have no corresponding specific marker and cannot be classified; on the other hand, the genetic variation information of single cell genomes is analyzed comprehensively and completely, and a plurality of control samples can be set, which greatly increases the accuracy of cell subgroup classification.

The single cell classification device disclosed herein can also have the following additional technical features: A data filtering module is suitable for performing data filtering based on the following criteria: when a plurality of pairs of duplicated paired-end reads are present, and the sequences of the plurality of pairs of reads are fully consistent, randomly selecting one pair of reads, and removing the other duplicated paired-end reads in said plurality of pairs of reads; and/or removing reads which are not uniquely aligned with the sequence of said reference genome.

A genotype determination module suitable for determining the consistent genotype of said each single cell through the following items: on the basis of said filtered reads, determining a possibility of a genotype of each single cell sample in a target region; on the basis of possibilities of genotypes of all the single cell samples in the target region, determining a pseudo-genome containing each site of all the samples; and selecting a genotype with a maximum probability from said pseudo-genome as the consistent genotype of each single cell sample.

A classification module suitable for removing at least one of the following items from the SNP dataset of said group to select an SNP site associated with cell mutation: non inter-group SNP sites, sites of loss of heterozygosity, and published SNP sites.

As described herein, the whole genome of at least one of said plurality of single cell samples is subjected to the whole genome amplification treatment before being sequenced, in which said classification module is suitable for removing sites that meet the following conditions, so as to remove sites of loss of heterozygosity: in samples that have not undergone whole genome amplification, the sequencing results being heterozygous sites; and in samples that have undergone whole genome amplification, at the same site, the number of samples with loss of heterozygous sites and data being greater than or equal to the number of the samples that have undergone whole genome amplification minus 3.

As described herein, the genotype file extraction module is suitable for screening said SNP dataset according to the following criteria: the quality value of the consistent genotype of each site being not less than 20, and the p value for the rank test being not less than 1%; and for SNPs of heterozygous variation: the major allele's sequencing quality value being not less than 20, and the sequencing depth being not less than 6, the minor allele's sequencing quality value being not less than 20, the sequencing depth being not less than 2, and the ratio of sequencing depths of two genotypes being within a range of 0.2-5.

Said classification module may be further suitable for extracting the information of each cell sample, and excluding contentious cells.

The device may comprise a screening module, said screening module being suitable for: determining the classified groups on the basis of the classification result, and calculating a statistic of all SNP sites of each gene in each class of groups, optionally performing a difference test on the obtained statistic to obtain a test value; and selecting a gene with the highest statistic or test value as a gene associated with cell mutation.

A gene screening as described herein, includes the following steps: classifying cells, so as to obtain the classified subgroups, and calculating a statistic of all SNP sites of each gene in each class of subgroups, optionally performing a difference test on the obtained statistic to obtain a test value; and selecting a gene with the highest statistic or test value as a gene associated with cell mutation. By pre-classifying cells, which may, e.g., according to pre-determined criteria, be divided into such as paracancerous cells and cancer cells, or other cell groups with known distinctions, and by statistically analyzing SNP sites in each class of groups, e.g., according to differences in the SNP type and distribution among differently classified groups, a gene closely associated with cell mutation can be effectively determined, and further by analyzing the function of the gene, a function closely associated with cell mutation can be determined, thereby determining the markers for cell mutation or specific status, such as diseases, of an organism body, e.g., a human, which include gene markers and functional markers. Methods that can be used for cell classification are not limited specifically, can be based on the clinical classification, and can also be the single cell classification method described previously. It needs to be noted that the term "subgroup" used herein is for being distinguished from the "group" in the single cell classification method, and on the premise of not affecting the understanding, sometimes the term "subgroup" herein is also directly referred to as "group".

Also as described herein, a gene screening device comprising: a computing unit, said computing unit being suitable for acquiring the classified subgroups according to the cell classification result, and calculating a statistic of all SNP sites of each gene in each class of groups, optionally performing a difference test on the obtained statistic to obtain a test value; and a sorting unit, said sorting unit sorting all genes according to the statistic or test value, and screening same to obtain a gene with the highest statistic or test value which is used as a gene associated with cell mutation.

Using the device can effectively implement the aforementioned gene screening method, and by pre-classifying cells, which may, e.g., according to pre-determined criteria, be divided into such as paracancerous cells and cancer cells, or other cell groups with known distinctions or with significant statistical differences, and by statistically analyzing SNP sites in each class of groups, e.g., according to differences in the SNP type or distribution among differently classified groups, a gene closely associated with cell mutation can be effectively determined, and further by analyzing the function of the gene, a function closely associated with cell mutation can be determined, thereby determining the markers for cell mutation or specific status, such as diseases, of an organism body, e.g., a human, which include gene markers and functional markers. The cell classification result can be implemented by the aforementioned single cell classification method. Thus, the gene screening device described herein may further comprise a cell classification device, and the cell classification device is the aforementioned single cell classification device, so as to classify cells to obtain the classified groups.

Thus, in view of the existing problems of existing single cell classification and screening methods, the present invention provides a single cell classification method and a device for implementing said methods in accordance with the claims.

The single cell classification method include the following steps:

aligning a result of reads of each single cell sample obtained by sequencing to the sequence of a reference genome, and performing data filtering on the alignment result;

according to filtered data, determining a consistent genotype of each single cell sample, and storing consistent genotypes of all the single cell samples as an SNP dataset;

extracting a genotype file on sites corresponding to positions in an SNP dataset of the reference genome from the stored SNP dataset;

and selecting an SNP site of cell mutation, and according to the genotype file on the SNP sites of cell mutation, classifying the cells.

A single cell classification device comprise:

a data filtering module, for aligning reads of each single cell sample obtained by sequencing to the sequence of a reference genome, and performing data filtering on the alignment result;

a genotype determination module, for determining a consistent genotype of each single cell sample according to filtered data, and storing consistent genotypes of all the single cell samples as an SNP dataset;

a genotype file extraction module, for extracting a genotype file on sites corresponding to positions in an SNP dataset of the reference genome from the stored SNP dataset;

and a classification module, for selecting an SNP site of cell mutation, and classifying the cells according to the genotype file on the SNPs of cell group mutation.

The single cell screening method described may include the following steps:

acquiring the starting and ending positions of genes in a predicted genome;

acquiring the classified groups according to a cell classification result, calculating a static of all SNP sites of each gene in each class of groups, and accumulating the statistic;

performing a difference test on the obtained statistic to obtain a test value;

and sorting predicted genes according to the statistic or test value, and screening same to obtain a gene with the highest statistic or test value.

A single cell screening device comprise:

an acquisition unit, for acquiring the starting and ending positions of genes in a predicted genome;

a computing unit, for acquiring the classified groups according to a cell classification result, calculating a statistic of all SNP sites of each gene in each class of groups, and accumulating the statistic; and performing a difference test on the obtained statistic to obtain a test value;

and a sorting unit, coupled to the acquisition unit and the computing unit, for sorting predicted genes according to the statistic or test value, and screening same to obtain a gene with the highest statistic or test value.

Next-generation sequencing (NGS) technology, is adopted herein through the bioinformatics methods, to analyze and study single cell genomes, and to collect cell subgroups (or microparticles) to perform subsequent in-depth studies. On one hand, the operation of labeling cells is avoided, which effectively solves the problem in traditional single cell classification methods that certain cell subgroups have no corresponding specific marker and cannot be classified; on the other hand, the genetic variation information of single cell genomes is analyzed comprehensively and completely, and a plurality of control samples are set, which greatly increases the accuracy of cell subgroup classification.

As described herein the single cell screening method, obtains cell subgroups (or microparticles) by screening, and increases the accuracy of cell screening.

The additional aspects and advantages of the present invention are set forth in the following detailed description, and with reference to the drawings in which:

## BRIEF DESCRIPTION OF THE DRAWINGS

- Figure 1 is a schematic view of repeated fragments (Duplication Reads) in the prior art;
- Figure 2 is a schematic view of fragments that are uniquely aligned onto a reference genome (Unique mapped reads) in the prior art;
- Figure 3 is a flow chart for the methods for single cell classification and screening;
- Figure 4 is an N-J relationship tree for renal cancer exome sequencing in the present invention;
- Figure 5 is a maximum likelihood relationship tree for renal cancer exome sequencing in the present invention;
- Figure 6 is a PCA result graph for renal cancer exome sequencing in the present invention, the abscissa representing the first principal component vector, and the ordinate representing the second principal component vector;
- Figure 7 is a PCA result graph for renal cancer exome sequencing in the present invention, the abscissa representing the first principal component vector, and the ordinate representing the third principal component vector;
- Figure 8 is a PCA result graph for renal cancer exome sequencing in the present invention, the abscissa representing the first principal component vector, and the ordinate representing the fourth principal component vector;
- Figure 9 is a Structure result chart for renal cancer exome sequencing in the present invention, in which "Series 1" represents a cancer cell group and "Series 2" represents a paracancerous cell group;
- Figure 10 is a schematic view of classification relationships of 53 cancer cells and 8 normal cells in the present invention;
- Figure 11 is a clustering schematic view of cancer cells and normal cells in the present invention, the abscissa representing the first principal component vector, and the ordinate representing the second principal component vector;
- Figure 12 is a schematic view of a single cell classification device;
- Figure 13 is a schematic view of a screening module.

## DETAILED DESCRIPTION OF THE INVENTION

The embodiments of the present invention are detailed below, and the examples of said embodiments are shown in the drawings, in which the same or similar marks represent the same or similar elements or elements with the same or similar functions from beginning to end. The embodiments described below by reference to the drawings are exemplary, only for explaining the present invention, and cannot be understood as limitations on the present invention.

The present invention adopts the next-generation sequencing (NGS) technology, through the bioinformatics methods, to analyze and study single cell genomes, and to screen and collect cell subgroups (or microparticles) to perform subsequent in-depth studies. Consequently, the present invention can be applied more efficiently and conveniently in clinical diagnoses and treatments (e.g., prenatal diagnosis, pre-implantation genetic diagnosis, individualized treatment, multi-point map production, sperm and egg typing, diagnosis of genetic diseases, tumor (e.g., lymphoma and leukemia) typing, and the like), medical researches (e.g., research into autism, nervous system diseases and autoimmune diseases, genomic mutation rate research, stem cell research, drug resistance research, drug target gene screening, transcriptome detection, cell model research, population identification and the like), archaeological researches and forensic detection.

The single cell samples involved in the present invention include nucleic acids (genomic DNA or RNA, e.g., non-coding RNA and mRNA); the single cells are derived from organism bodies and prepared using conventional methods. Particularly, the DNA or RNA can be obtained by extraction or amplification from bacteria, protozoa, fungi, viruses and single cells of higher organisms/higher plants and animals, e.g., mammals, and in particular, humans. The single cells can be obtained by in vitro culture, or direct separation from clinical samples (including plasma, serum, spinal fluid, bone marrow, lymph fluid, ascites, pleural effusion, oral liquid, skin tissue, the respiratory tract, the digestive tract, the genital tract, the urinary tract, tears, saliva, blood cells, stem cells and tumors), and fetal cells can be derived from embryos (e.g., one or more embryoid bodies/embryos) or maternal blood, and can be derived from living or dead organism bodies. The samples include single cell suspensions, paraffin-embedded tissue sections and puncture biopsy tissues.

The samples can reflect the specific status of cells, e.g., cell proliferation, cell differentiation, cell apoptosis/death, disease status, external stimulation status and developmental stages.

Particularly, the single cell samples are obtained from mammals, including preimplantation embryos, stem cells, suspected cancer cells and pathogenic organisms, and even obtained from crime scenes. For instance, the analysis of human blastomere cells (an embryo at eight-cell stage and after this stage) can determine whether a genetic deficiency occurs or not in the genome of the fetus.

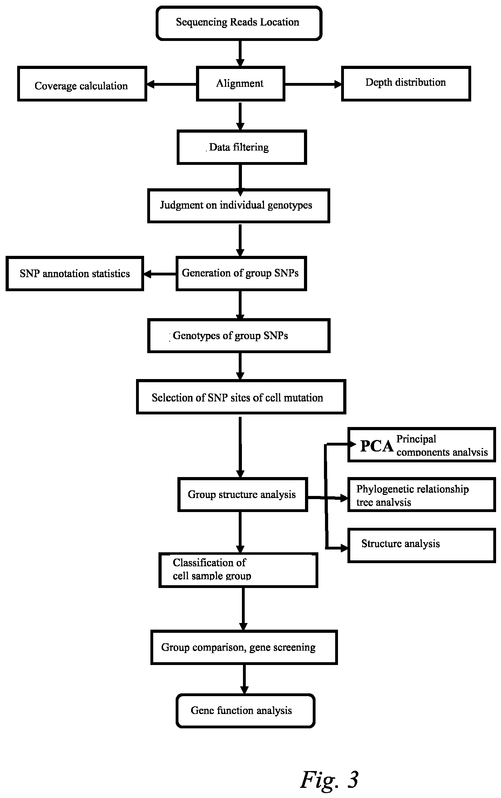

The specific implementation process for the single cell classification method in the present invention will be detailed hereinafter in conjunction with Figure 3. In that, Figure 3 shows the flow process starting from (7).

(1) Single cell separation: physical and mechanical, chemical and biological methods, e.g., microfluidics, mouth suction separation, gradient dilution, low melting point agarose fixation and other methods, are used to perform separation to obtain single cells containing complete genomes.

(2) Cell lysis: for the single cells obtained by separation, the detergent method, the boiling method, the alkaline denaturation method, the lysozyme method, the organic solvent method and other methods are used to lyse nuclei to obtain complete genomic DNA of the cells.

(3) Single cell whole genome amplification (WGA):

Currently, there are 2 strategies for whole genome amplification: i.e., PCR-based amplification, e.g., DOP-PCR, PEP-PCR and T-PCR, and linear DNA amplification, e.g., OmniPlex WGA and multiple displacement amplification (MDA). The single cell whole genome amplification is performed to meet the starting DNA amount required by the next-generation sequencing technology.

(4) Quantification of whole genome amplification products: the gel electrophoresis detection, Agilent 2100 Bioanalyzer detection, Quant-iT™ dsDNA BR Assay Kit detection and other methods are used to quantify the amplification products of whole genomes of single cells, and only for a sample with a result showing no degradation and meeting the starting DNA amount required by the next-generation sequencing technology, DNA library construction and sequencing on a machine can be continued.

(5) Detection of the whole genome amplification products: STR detection, Housekeeping Gene detection and other methods are used to detect the single cell whole genome amplification products, and only for a sample with a result showing that the amplification products are uniformly distributed on the chromosomes of the corresponding species, DNA library construction and sequencing on a machine can be continued.

(6) DNA library construction and sequencing on a machine: the conventional whole genome DNA library construction or exome sequence capture technology is used to perform DNA library construction, and after the quality inspection is passed, single cell genomes are sequenced using the next-generation sequencing technology, e.g., the Illumina HiSeq 2000 sequencing system, the Illumina Genome Analyzer II sequencing system, the AB SOLiD™ 4.0 sequencing system, the Roche GS FLX Titanium System and the like.

(7) Location of Reads

A result of Reads of each single cell sample obtained by sequencing is aligned to the sequence of a reference genome (e.g., human genomes HG18 and HG19) using short-reads alignment software (e.g., SOAPaligner, BWA and Bowtie), and according to the specific data, optimal parameters are set to accurately locate the Reads.

(8) Basic data statistics

According to the above-mentioned alignment result, the sequencing depth and coverage and other results of each sample (a single cell or tissue) relative to the sequence of the reference genome are calculated.

The sequencing depth means a mean depth at which a genome is sequenced, and the calculation method is dividing the total base number of sequencing by the size of the genome.

The sequencing coverage means an approximate proportion the sequenced part in a genome accounts for, and the calculation method is dividing the covered sites of the genome by the effective length of the genome.

The sequencing depth and coverage are used to evaluate whether the amount of data used for bioinformatics analysis is enough or not and whether the sequencing is uniform or not.

(9) Data filtering

When a plurality of pairs of duplicated paired-end reads are present, and the sequences of the plurality of pairs of reads are fully consistent, one pair of reads are selected randomly, and the other duplicated paired-end reads in said plurality of pairs of reads are removed; and/or reads which are not uniquely aligned onto the sequence of the reference genome are removed.

According to the characteristics of data, duplicated paired-end (pair-end) reads from each DNA library are selected, e.g., duplicated paired-end reads caused by excessive amplification times, of course without limitation to PCR, they can also be caused by other amplification modes.

When a plurality of pairs of duplicated paired-end reads are present, and the sequences of said duplicated paired-end reads are fully consistent, one pair are selected randomly there from, and the other duplicated paired-end reads are removed.

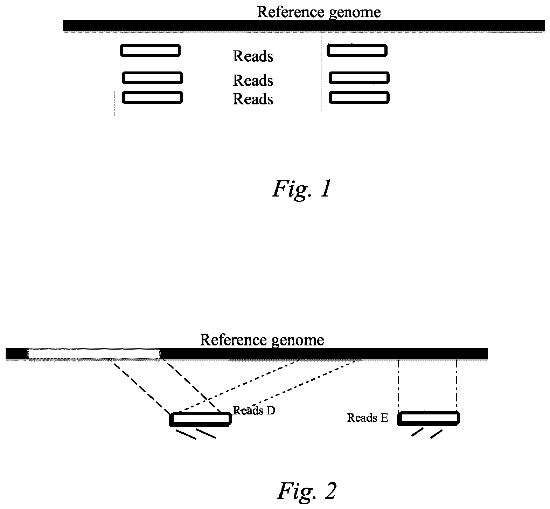

As shown in Figure 1, the sequences of the three pairs of reads, A, B and C, are fully consistent, the matched starting and ending positions to the genome are also fully consistent, and the starting and ending positions are fully consistent, thus forming duplicated paired-ends. In this case, only one pair of reads therein is retained randomly, and the other repeated reads are removed.

For confirming the accuracy of the data, reads which are not uniquely aligned with the sequence of the reference genome can also be removed. The exome sequencing on the human genome is taken as an example, of course, without limitation to this, e.g., other mammals and the like may be sequenced, and the sequencing mode is also not limited to the exome sequencing, e.g., whole genome sequencing and other modes. Considering that there cannot be a plurality of copies for the exon regions of a human on the genome, i.e., repeated sequences are impossible, reads obtained by exome sequencing should mostly be uniquely aligned with a human reference genome. For excluding influences caused by mismatches, only reads which are uniquely aligned with the reference genome are selected to be analyzed (i.e., reads with the hit number of 1), so that the influences caused by mismatches are reduced to a great extent.

As shown in Figure 2, Reads D is aligned with a plurality of positions on the reference genome, while Reads E is only aligned with a sole position, and since the exome on the genome are not repeated regions, Reads D is removed directly.

(10) Judgment on individual genotype

We take full consideration and use of existing information on the reference genome, use genotype judgment software (e.g., SOAPsnp, SAMtools/Pileup/Mpileup), and judge a possible genotype of each cell sample in a target region to obtain a file on the possibility of genotypes for each cell sample.

In the present invention, it is the data of exon regions that are detected, and in this embodiment, the target region is a region where an exon locates. Generally, specific regions to be sequenced and analyzed by bioinformatics will be pointed out, such as:

| chr1 | 20038 | 20358 |
| chr1 | 58832 | 59992 |
| chr1 | 357410 | 358570 |

(11) SNP dataset

Since some regions of low depth exist in the genome of each cell, the present invention synthesize the files on the possibility of genotypes for all cells, uses the maximum likelihood approach to integrate the data of all the cells, and produces a pseudo-genome containing each site of all samples. A genotype with a maximum probability is selected as a consistent genotype of each cell, and high-quality SNPs are detected through the genotype, the sequencing depth and other information. After consistent sequences of the samples are obtained, the result is stored as an SNP dataset in an group SNPs format.

(12) Genotypes of group SNPs

According to position information in an SNP dataset of the reference genome, a genotype at a corresponding site of each cell is extracted from the file on the possibility of genotypes to obtain a genotype file on group SNPs at the corresponding sites of the various cells. A site indicates the position where an SNP locates.

(13) Selection of SNP sites associated with cell mutation

The present invention mainly lies in seeking differential sites among various cells, and thus, sites associated with cell mutation must be selected.

Firstly, non inter-group SNP sites are removed.

The definition of a non inter-group SNP site: the base types of all individuals are identical, and all are SNPs relative to a reference sequence, and then the site is a non inter-group SNP site.

For instance, the reference sequence is A, there is a heterozygous base type R at the site for all individuals, and the site is a non inter-group SNP site. For instance

chrl 319660 RRRRRRRRRRRRRR

Secondly, sites of loss of heterozygosity can also be removed. Because during WGA amplification on a single cell, a condition exists that only one chromosome in a pair of chromosomes is amplified, which result in allele dropout, there exists a phenomenon of loss of heterozygosity at certain sites for each detected cell. The disturbance by this class of sites is excluded.

And finally, published SNP sites are removed, e.g., taking humans as an example, normal human SNP sites are removed, i.e., the dbSNPs of the human genome HG18, the SNPs of Yanhuang No. 1 and the SNPs of the 1000 Genomes are removed.

The is no particular order for the three above-mentioned operations, and after the execution of these three operations is finished, obtained SNP sites are SNP sites associated with cell mutation.

(14) Group structure analysis

According to the genotype file on the SNP sites of cell group mutation, the cells are classified using the commonly used methods in group analysis by bioinformatics, respectively, e.g., tree construction by the neighbor-joining (N-J) method, MEGA software, principal components analysis (PCA), group structure and the like. When the cells are being classified, at least one of the above methods can be used. As one embodiment of the present invention, all the above methods are used, and when the classification results of the various methods are consistent, same is confirmed as the final cell classification result.

14-1. Tree construction by the neighbor-joining (N-J) method

Because the degrees of the selection to which different categories of cells would be subjected are different, i.e., the single-base mutation rates are different, the differences among categories in evolution are also shown in SNPs. The degree of difference between two cells can be obtained by calculation with SNP data. Through theoretical calculations, the formula is obtained as follows:

$$Dis_{ij} = \sum_{k=1}^{n} diff_{ij}^{k}$$

In the above formula, $Dis_{ij}$ represents the differential distance between Cell $i$ and Cell $j$, where $n$ is the total number of SNPs, and $diff_{ij}^{k}$ represents the degree of difference between Cell $i$ and Cell $j$ at Site $k$, where the definition is

$$diff_{ij}^{k} = \begin{cases} 0 & \text{the genotypes are exactly the same, e.g., at Location k, Cell } i\colon A, j\colon A \\ 1 & \text{the genotypes are completely different, e.g., at Location k, Cell } i\colon A, j\colon C \\ 0.5 & \text{the genotypes are partly different, e.g., at Location k, Cell } i\colon A, j\colon M \end{cases}$$

Since the human genome is diploid, A represents the two sites of alleles are both A, and $M$ is a heterozygous site, i.e., a combination of A and C. Based on the genotyping file on the SNP sites of cell group mutation obtained by the above-mentioned step (13), statistics is performed on the differences by comparison between any two of all single cell samples to obtain a difference matrix by comparison between any two in a target region, the above-mentioned difference matrix is applied to the Fneighbor program (emboss.bioinformatics.nl/cgi-bin/emboss/help/fneighbor), and the program can obtain a phylogenetic tree thereof through the neighbor-joining (N-J) method.

14-2. MEGA software

The MEGA software (www.megasoftware.net) is used to make the genotype file on all SNP sites of each cell form a sequence, with one cell corresponding to one sequence, as an input file for MEGA, and MEGA constructs a relationship tree according to the differences among various cells in sequence, in which the software has three construction methods (Maximum likelihood, Least Squares and Maximum parsimony).

14-3. Principal components analysis (PCA)

In statistics, the principal components analysis (PCA) is a technology for simplifying datasets, and is a linear transformation. This transformation transforms data into a new coordinate system, so that for any data projection, the first large variable number locates on the first coordinate (referred to as the first principle component), the second large variable number locates on the second coordinate (the second principle component), and so on. The principal components analysis is often used for reducing the number of dimensions of a dataset, and at the same time, it retains a characteristic variable with the maximum contribution to the dataset. Same is realized by retaining low-order principal components and ignoring high-order principle components. This is because the low-order principal components can often retain the most important aspect in the dataset.

According to the reference document, A tutorial on Principal Components Analysis. Lindsay I Smith, 2002-02, and the characteristics of real SNP data, firstly, the SNP data are converted into a digital matrix (0 for what is consistent with the reference sequence, 2 for what is the contrary, and 1 for a degenerate base) and homogenized. Then a linear vector equation is constructed through the above-mentioned methods introduced.

$$y_i = a_{i0} + a_{i1}x_i + a_{i2}x_i^2 + .... + a_{i20}x_i^{21}$$

Where $i$ is from 1 to 21 and represents the $i$th sample.

The R language software package's powerful ability to solve equations is applied to solve same to obtain a matrix $a$, and according to the characteristics of the data of various cells, the first four principle component vectors are extracted and the vectors are used as coordinate axes to display the clustering conditions of various cells.

14-4. Group structure by Structure

The Structure software (pritch.bsd.uchicago.edu/software/structure2 _1. html), based on genotyping data of SNP sites, deduces whether different groups exist or not, and judges the group each individual belongs to. According to the instructions of the software, the genotype file on group SNPs is subjected to format conversion and used as an input file for Structure, and up to 50 thousand simulations are adopted in a mixed model; and when the existence of a plurality of groups is assumed, the probabilities of each cell belonging to various classes of groups are calculated.

Through the flow process of the above method, the classification of single cells is realized. Based on the classification, single cell screening can also be further performed, and the flow process is as follows:

(15) Group structure analysis results

According to results of the above-mentioned group structure analyses, the classification of single cells is realized, the information of each cell sample is extracted, and contentious cells, e.g., unclearly classified or evident outlier samples, are excluded.

(16) Relevant gene screening

According to SNPs of cell groups, comparisons of these groups in genome are performed through a series of statistics and tests, regions or genes with a significant difference are found out, and genes with a relatively high correlation coefficient can be obtained by screening.

Taking the human genome as an example, the specific approach is as follows:
The annotation file corresponding to HG18 is downloaded from the human genome data base to obtain the starting and ending positions of over 30,000 genes in the human genome predicted currently.

According to the cell classification result, the classified groups are obtained, a static of all SNP sites of each gene in each class of groups is calculated, and the statistic is accumulated. Said each gene here means genes in the predicted genome.

In that, the mainly adopted formula for calculating the statistic $\pi$ is as follows, $\pi$ is an index for measuring the level of polymorphism of a group, a and b mean the numbers of samples of two bases in a certain group, and the formula can be:

$$\pi = \frac{a * b}{C_{a+b}^2}$$

Difference test can also be performed on the obtained statistic to obtain a test value. The adopted test value is at least one of *Lod, Fst* and *Pbs.* As one embodiment of the present invention, the three above test values can be adopted, and when the three above test values are consistent, same is used as the final test value result.

These over 30,000 genes are sorted according to the statistic and/or test value, and a gene with the highest statistic and/or test value is selected. That is to say, same can be sorted according to the statistic, can be sorted according to the test value, and can also be sorted according to the statistic and test value. As one example, the last method can be adopted, and when the sorting result obtained according to the statistic is consistent with the sorting result obtained according to the test value, the gene is used as the final gene obtained by screening.

(17) Gene function analysis

The functions of genes obtained by screening are examined, and functional analyses are performed, respectively. It is judged whether these genes are affected in certain pathways and then associated with the pathogenesis of certain diseases.

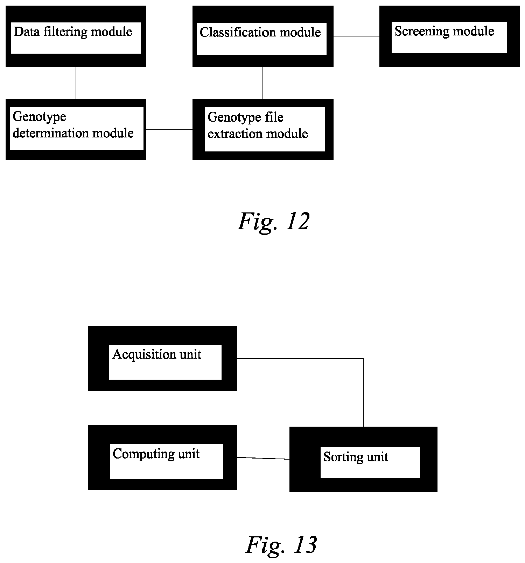

As shown, Figure 12 is a schematic view of the single cell classification device in the present invention. The device comprises:

a data filtering module, for aligning a result of reads of each single cell sample obtained by sequencing to the sequence of a reference genome, and performing data filtering on the alignment result;
a genotype determination module, coupled to the data filtering module, for determining a consistent genotype of each single cell sample according to filtered data, and storing consistent genotypes of all the single cell samples as an SNP dataset;
a genotype file extraction module, coupled to the genotype determination module, for extracting a genotype file on sites corresponding to positions in an SNP dataset of the reference genome from the stored SNP dataset;
and a classification module, coupled to the genotype file extraction module, for selecting an SNP site associated with cell mutation, and classifying the cells according to the genotype file on SNPs of cell group mutation, the adopted classification method including at least one of the tree construction by the neighbor-joining (N-J) method, the MEGA software, the principal components analysis (PCA) and the group structure by Structure.

Also shown as Figure 12, the single cell classification device may comprise:
a screening module, coupled to the classification module, for acquiring the starting and ending positions of genes in a predicted genome; according to the classification result, acquiring the classified groups, calculating a statistic of all SNP sites of each gene in each class of groups, and accumulating the statistic; performing a difference test on the obtained statistic to obtain a test value; and sorting predicted genes according to the statistic or test value, and screening same to obtain a gene with the highest statistic or test value.

The screening module can further comprise the following units shown as Figure 13:

an acquisition unit, for acquiring the starting and ending positions of genes in a predicted genome;
a computing unit, for acquiring the classified groups according to the cell classification result, calculating a statistic of all SNP sites of each gene in each class of groups, and accumulating the statistic; and performing a difference test on the obtained statistic to obtain a test value;
and a sorting unit, coupled to the acquisition unit and the computing unit, for sorting predicted genes according to the statistic or test value, and screening same to obtain a gene with the highest statistic or test value.

The specific operations executed by the various modules in the single cell classification device in accordance with the claims are reflected in the flow process of the above-mentioned methods, and the specific operations of the various modules can also be found according to the following embodiments.

The scheme of the present invention will be explained below in conjunction with the embodiments. A person skilled in the art would understand that the following embodiments are only used to illustrate the present invention and should not be regarded as limitations on the scope of the present invention. Where the specific techniques or conditions are not noted in the embodiments, the embodiments should be performed according to the techniques or conditions described in the literature in the art (e.g. referring to Molecular Cloning: A Laboratory Manual, 3rd edition, Science Press, written by J. Sambrook, et al. and translated by Huang Peitang, et al.) or according to product instructions. The reagents or instruments used with no manufacturer noted are all conventional products that can be obtained by purchase in the market, e.g., can be purchased from Illumina Corporation.

**Embodiment 1: single renal cancer cell classification**

1-1. Location of Reads

The result of Reads of each single cell sample obtained by sequencing was aligned to the sequence of a reference genome (the human genome HG18) with the SOAPaligner alignmetn software (soap.genomics.org.cn/soapaligner.html). Since human SNPs were two thousandths and the length of Reads was 100 bp, during the alignment by SOAP, the parameters were set that each piece of Reads had a maximum of 3 mismatches and Gap was not allowed, so as to ensure that the positions of Reads that could be mapped on were accurate.

1-2. Basic data statistics

According to the result of the above-mentioned alignment, the sequencing depth and coverage and other results were calculated for each sample (a single cell or tissue) relative to the sequence of the reference genome, and when whole genome sequencing was obtained by statistics and the mean depth was around $3\times$, due to the presence of certain bias of PCR amplification, the coverage of the samples was of relatively large fluctuation between 55%-90%.

Table 1 The whole genome sequencing coverage and depth data of single renal cancer cell samples

| Single cell sample ID | Coverage | Mean depth | Single cell sample ID | Coverage | Mean depth |
|---|---|---|---|---|---|
| RC-1 | 83.24% | 2.97 | RC-12 | 64.75% | 2.54 |
| RC-2 | 66.69% | 2.66 | RC-13 | 54.37% | 2.78 |
| RC-3 | 62.43% | 2.94 | RC-14 | 67.36% | 2.69 |
| RC-4 | 67.18% | 2.68 | RC-15 | 61.15% | 2.88 |
| RC-5 | 72.12% | 3.06 | RN-1 | 83.38% | 2.61 |
| RC-6 | 84.21% | 3.04 | RN-2 | 78.38% | 2.44 |
| RC-7 | 79.23% | 3.20 | RN-3 | 64.56% | 2.53 |
| RC-8 | 75.01% | 3.10 | RN-4 | 66.18% | 2.84 |
| RC-9 | 62.72% | 3.21 | RN-5 | 82.99% | 2.92 |
| RC-10 | 61.07% | 2.87 | RN-T | 88.12% | 2.71 |
| RC-11 | 59.66% | 2.84 | | | |

In that, RC-1 to RC-15 represent single renal cancer cells, totally 15 single cell samples; RN-1 to RN-5 represent single paracancerous cells; and RN-T represents a normal tissue from which DNA was directly extracted to be sequenced, so as to be used as a control for data analysis and assessment. The single paracancerous cells were mainly used as the control samples. There also existed that the single paracancerous cells and the normal tissue were both used as the control samples at the same time, e.g., during the removal of sites of loss of heterozygosity, the two above-mentioned control samples were used.

In exome sequencing, the sequencing depth was increased, and when the mean depth of a target exon region was around $30\times$, the coverage of the target region reached 80%-96%. In a statistical sense, for one site supported by four pieces of reads, it could be judged that the accuracy of the bases of the site reached 99%; moreover, sites with the sequencing depth of 4 obtained by statistics accounted for a proportion reaching 60%-90% of exon regions, indicating that data of exome sequencing was better than data obtained by whole genome sequencing.

Table 2 The exome sequencing coverage and depth data of single renal cancer cell samples

| Single cell sample name | Coverage | Coverage (sequencing depth >= 4) | Mean depth | Single cell sample name | Coverage | Coverage (sequencing depth >= 4) | Mean sequencing depth |
|---|---|---|---|---|---|---|---|
| RC-1 | 95.84% | 89.10% | 34.63 | RC-11 | 81.74% | 62.57% | 27.59 |
| RC-2 | 90.92% | 76.83% | 33.82 | RC-12 | 91.73% | 78.00% | 36.06 |
| RC-3 | 86.81% | 71.26% | 36.42 | RC-13 | 81.65% | 64.14% | 30.40 |
| RC-4 | 89.36% | 72.58% | 24.99 | RC-14 | 89.36% | 71.93% | 23.91 |
| RC-5 | 92.84% | 79.55% | 32.63 | RC-15 | 87.74% | 74.25% | 51.74 |
| RC-6 | 95.86% | 88.54% | 32.56 | RN-1 | 94.74% | 86.04% | 25.85 |
| RC-7 | 95.37% | 87.09% | 41.03 | RN-2 | 95.52% | 86.80% | 27.45 |
| RC-8 | 92.51% | 78.72% | 27.29 | RN-3 | 90.84% | 79.25% | 37.41 |
| RC-9 | 82.71% | 65.58% | 32.13 | RN-4 | 89.95% | 74.00% | 32.65 |
| RC-10 | 81.77% | 62.49% | 24.72 | RN-5 | 96.05% | 88.42% | 31.56 |
| | | | | RN-T | 95.73% | 87.90% | 32.97 |

By comparison of the 2 above-mentioned tables, it can be seen that the depth of whole genome sequencing was too low to perform subsequent analyses; however, the depth of exome sequencing was higher. In addition, taking the cost issues of sequencing into account, the analysis was performed below mainly based on the data obtained by exome sequencing.

1-3. Data filtering

According to the characteristics of data, duplicated paired-end reads caused by an excessive number of amplification times were selected from each DNA library, and when the sequences of a plurality of pairs of duplicated paired-end reads were fully consistent, one pair of reads were selected randomly, and the other paired reads were removed.

For instance, the sequences of the three pairs of reads, A, B and C, in Figure 1 are fully consistent, and the matched starting and ending positions on the genome are also fully consistent. In this case, only one paired reads therein is retained randomly.

For confirming the accuracy of the data, considering that there cannot be a plurality of copies for the exon regions of a human on the genome, i.e., the repeated sequences are impossible, reads obtained by exome sequencing should mostly be uniquely aligned with the human reference genome. For excluding influences caused by mismatches, only reads which are uniquely aligned with the reference genome were selected to be analyzed (i.e., reads with the hit number of 1), so that the influences caused by mismatches were reduced to a great extent.

As shown in Figure 2, Reads D is aligned with a plurality of positions on the reference genome and Reads E is only aligned with a sole position, and since exons are not repeated regions on a genome, Reads D is directly removed.

1-4. Judgment on individual genotype

We took full consideration and use of existing information on the human genome (as the reference genome in the embodiment), downloaded dbsnps corresponding to the human genome (HG18) from the NCBI website, used same as the priori probability of SOAPsnp, and on the basis of the study results determined currently, set the priori probability of a heterozygous site SNP to be 0.0010, and the priori probability of a homozygous site SNP to be 0.0005.

After the above parameters were set, filtered data of steps 1-3 were inputted into the SOAPsnp software, and the filtered data were aligned to the reference genome by the SOAPsnp software to obtain an alignment result which is a CNS file.

1-5. SNP dataset

Because there exists some regions of low depth in the genome of each cell, the present invention synthesized the files on the possibility of genotypes for all cells, used the maximum likelihood approach to integrate the data of all cells, and produced a pseudo-genome containing each site of all samples. A genotype with a maximum probability was selected as a consistent genotype of each cell, and high-quality SNPs were detected through the genotype and depth and other information. After a consistent sequence of a sample was obtained, the result was stored in the group SNPs format.

1-6. SNP genotype

According to position information in the SNP dataset of the reference genome, a corresponding site of a genotype of each cell was extracted from the CNS file to obtain a genotype file on group SNPs at the corresponding sites of the various cells. The format of the file is shown as Table 3.

The "SNP position" represents the position of the SNP site on a chromosome, the "base type" corresponds to a base type of the genome of a certain cell at this site, and sites with the depth of 0 is represented as "-" (i.e., sites with data lost). The "sample ID" corresponds to 21 single cell or tissue DNA samples.

Table 3 Schematic format of a genotype file on group SNPs at the corresponding sites of various cells

| Sample ID / Base type / SNP position | RC-1 | RC-2 | RC-3 | RC-4 | RC-5 | RC-6 | RC-7 | RC-8 | RC-9 | RC-10 | RC-11 | RC-12 | RC-13 | RC-14 | RC-15 | RN-1 | RN-2 | RN-3 | RN-4 | RN-5 | RN-T |
|---|---|---|---|---|---|---|---|---|---|---|---|---|---|---|---|---|---|---|---|---|---|
| chr19 10079226 | S | S | S | S | C | S | S | S | S | S | C | S | S | S | C | C | S | C | C | S | S |
| chr19 10079332 | R | R | R | R | R | R | R | G | R | R | R | R | R | R | R | R | R | R | R | R | R |
| chr19 10079408 | R | R | R | R | R | R | R | G | R | R | R | R | R | R | R | R | R | R | R | R | R |
| chr19 10082680 | C | C | C | C | C | C | Y | C | C | C | C | C | C | C | C | C | C | C | C | C | C |
| chr19 10083195 | G | R | R | A | G | R | R | R | R | G | R | R | G | R | G | R | R | R | - | R | A |

The number of group SNPs of the 21 single cell or tissue DNA samples relative to the human genome HG18 in the target region was 93957. In that, combinations of heterozygous sites are represented by the following letters: "M" represents "A and C", "R" represents "A and G", "W" represents "A and T", "Y" represents "C and T", "S" represents "C and G" and "K" represents "G and T".

1-7. Selection of SNP sites associated with cell mutation

The present invention mainly lies in seeking differential sites among various cells, thus sites associated with cell mutation must be selected.

Table 4 Schematic non inter-group SNP sites

| Sample ID / Base type / SNP position | RC-1 | RC-2 | RC-3 | RC-4 | RC-5 | RC-6 | RC-7 | RC-8 | RC-9 | RC-10 | RC-11 | RC-12 | RC-13 | RC-14 | RC-15 | RN-1 | RN-2 | RN-3 | RN-4 | RN-5 | RN-T |
|---|---|---|---|---|---|---|---|---|---|---|---|---|---|---|---|---|---|---|---|---|---|
| chr1 10402265 | R | R | R | R | R | R | R | R | - | R | R | R | R | R | R | R | R | R | R | R | R |
| chr1 11001664 | R | R | R | R | R | R | R | R | R | R | R | R | R | R | R | R | R | R | R | R | R |
| chr1 12775804 | W | W | W | W | W | W | W | W | W | W | W | W | W | W | W | W | W | W | W | W | W |
| chr1 12775818 | Y | Y | Y | Y | Y | Y | Y | Y | Y | Y | Y | Y | Y | Y | Y | Y | Y | Y | Y | Y | Y |

Firstly, non inter-group SNP sites were removed, and these sites are shown as Table 4. The base types of the genomes of all single cells at one and the same site were consistent, that is to say, the group constituted by the 21 cells was consistent at the site. By calculation, there were 504 such sites, after the removal of which 93,453 SNP sites remained.

Secondly, since during WGA amplification on a single cell, there exists the case that only one chromosome in a pair of same is amplified, which causes allele dropout, i.e., for an originally heterozygous site, only one base type therein is detected during sequencing, there exists the phenomenon of loss of heterozygosity at certain sites in each cell detected, such as the sites shown in Table 3:

```
>
chr19    10079332    R R R R R R R G R R R R R R R R R R
chr19    10079408    R R R R R R R G R R R R R R R R R R R
```

That is, the 9th single cell sample RC-9 is originally very likely to be a heterozygous site at the site; however, since only one base type is amplified, the site is judged as a homozygous site.

For excluding the disturbance by this class of sites, taking into consideration that the probability that the loss of heterozygosity occurs in a plurality of samples at one and the same site at the same time is extremely low, the present invention adopted the following strategy:

First, RN-T (i.e., the final column) that was a normal tissue from which DNA was directly extracted to be sequenced must be a heterozygous site. That is because this sample was not subjected to WGA amplification and the loss of heterozygosity could not occur.

Second, in the other 20 single cell samples, the number of samples of heterozygous sites and those with data lost was greater than or equal to 18. That is to say, the loss of heterozygosity was allowed to occur in only at most two single cell samples at one and the same site. That is because the probability that the loss of heterozygosity occurrs in three or more samples at one and the same site at the same time is extremely low.

The two above-mentioned conditions were required to be both met, i.e., the final column that was a normal tissue from which DNA was directly extracted to be sequenced was a heterozygous site, and in the other 20 single cell samples, the number of samples of heterozygous sites and those with data lost was greater than or equal to 18. Only such sites were removed.

By calculation, there were a total of 3,975 such sites, and the number of SNPs obtained by this filtering step was 89,478.

Finally, in order to obtain sites related to single renal cancer cell mutation, published normal human SNP sites need be removed, i.e., dbSNPs of the human genome HG18, SNPs of Yanhuang No. 1 and SNPs of the 1000 Genomes were removed, and 50,524 SNP sites associated with mutations of the various cells were obtained.

1-8. Group structure analysis

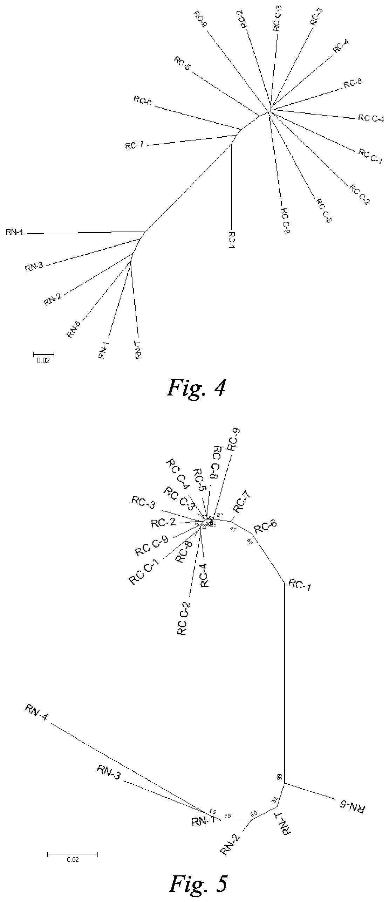

According to the genotype file on the SNP sites of cell group mutation, the cells were classified using the commonly used methods in group analysis by bioinformatics, respectively. The classification is determined by branches and clustering conditions of a phylogenetic tree. As shown in Figure 4, RC and RN were obviously clustered into two separate parts in the phylogenetic tree, and thus were divided into two classes.

1-9.1. Tree construction by the neighbor-joining (N-J) method

As shown in Figure 4, the cells could be classified according to the phylogenetic tree.

1-9.2. MEGA software

Figure 5 is a relationship tree constructed by the maximum likelihood approach, and the cells were classified according to the relationship tree.

1-9.3. Principal components analysis (PCA)

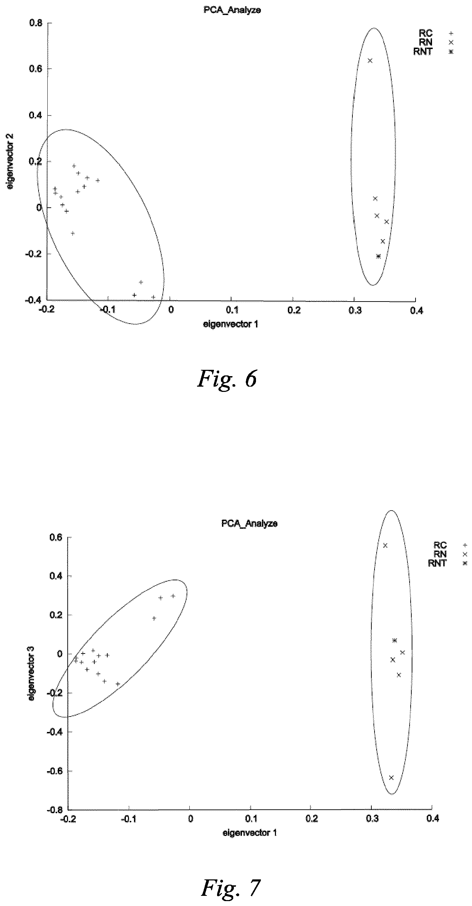

With renal cancer exome sequencing PCA results shown as Figure 6, Figure 7 and Figure 8, the cells were classified according to the clustering conditions.

1-9.4. Group structure by Structure

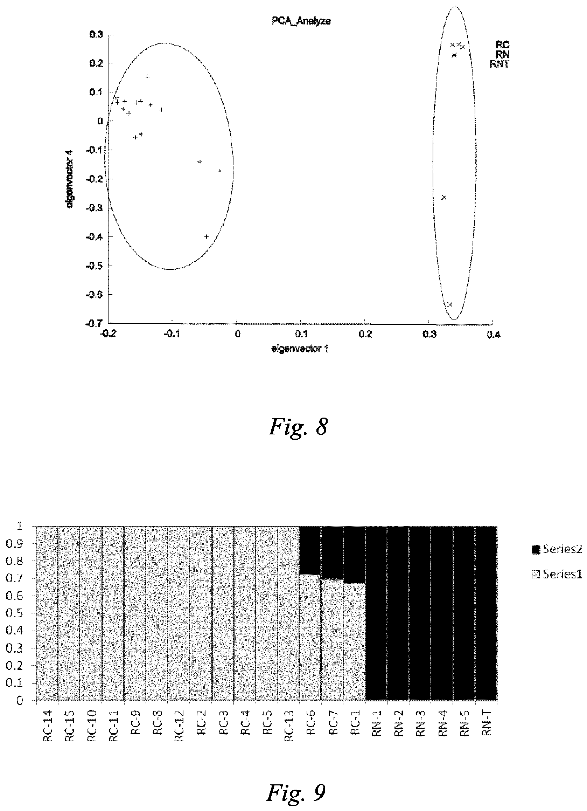

As in Figure 9, the abscissa represents the sample name, the ordinate represents the probability of a group each sample belongs to, and the single cells were classified according to the probabilities. As shown by Figure 9, the 20 single cells could roughly be divided into two groups. The Structure result of renal cancer exome sequencing is shown as Figure 9.

1-10. Group structure analysis results

According to results of the above-mentioned group structure analyses, the information of each cell sample was extracted, and contentious cells were excluded (unclearly classified or evident outlier samples). From the results of the various above-mentioned group structure analyses, the sampling was normal and the classification was reasonable. These 20 single cell samples could roughly be divided into 2 groups, i.e., a cancer cell group (15 RCs) and a paracancerous cell group (5 RNs), in which RC-1, RC-6 and RC-7 were a subgroup of cancer cells.

The information of cell samples means what are cancer cells and what are paracancerous cells in the single cells being analyzed (determined during sampling), and the information of cell samples was only used as a reference and needed to be analyzed together with the clustering results. If during sampling, the information of cell samples considers the cells to be cancer cells and paracancerous cells, and during clustering, same are exactly divided into two clustered groups, the results are demonstrated to be corresponding to each other; and if the information of cell samples during sampling is not consistent with the clustering results, the clustering results prevail.

As further clustered together in cancer cell clustering, RC-1, RC-6 and RC-7 were confirmed as a subgroup of cancer cells.

1-11. Screening of genes related to renal cancer

According to the SNPs of the above-mentioned two cell groups, RC and RN, in exon regions, these two groups were compared in the exon regions through a series of statistics and tests, regions or genes with significant differences were found out, and genes with a relatively high correlation coefficient to the case with renal cancer could be obtained by screening. The specific approach was as follows:

1-11.1 The annotation file corresponding to HG18 was downloaded from the human genome data base to obtain the starting and ending positions of over 30,000 genes predicted currently in the human genome.

1-11.2 According to the cell classification result, two groups, RC and RN, were obtained, a static of all SNP sites of each gene in each class of groups was calculated, and said statistic was accumulated.

In that, the mainly adopted formula for calculating the statistic $\pi$ was as follows, $\pi$ was an index for measuring the level of polymorphism of a group, a and b meant the numbers of samples of two bases in a certain group, and the formula could be:

$$\pi = \frac{a*b}{C_{a+b}^2}$$

As in the above-mentioned 15 RC samples, there were a total of 30 chromosomes, and for the following two sites: Site 1 was C on only 1 chromosome, and was T on the other 29 chromosomes (a = 1, b = 29); Site 2 was C on 15 chromosomes, and was T on the other 15 chromosomes (a = 15, b = 15). After they were substituted into the formula, it could be obtained that the $\pi$ value for Site 1 was 0.06 and the $\pi$ value for Site 2 was 0.517, and thus there was a significant difference between these 2 sites in polymorphism.

During the statistics on the polymorphism of a gene, the $\pi$ values for all sites of the gene were accumulated, and the $\pi$ value for a non SNP site was 0 (when $a = 0$ or $b = 0$, $\pi = 0$), that is, for a certain group, the $\pi$ values for all SNP sites of the gene were accumulated.

$$Pi_{Gene} = \sum_{SnpInGene} \pi$$

1-11.3 These over 30,000 genes were sorted according to the statistic or test value, genes with the highest statistics or test values was selected, and the functions of these genes were examined.

The adopted test value was at least one of the following test values: *Lod, Fst,* and *Pbs,* and the embodiment adopted the above three test values. The various test values and the calculation processes are specifically illustrated below.

Data were substituted for these two groups, RC and RN, respectively, and $Pi^{RC}_{Gene}$ and $Pi^{RN}_{Gene}$ could be obtained. Because it was needed to obtain the differences between these two groups by comparison, *Lod* was defined as follows:

$$Lod_{Gene} = 1 - (Pi^{RC}_{Gene} / Pi^{RN}_{Gene})$$

If the difference between $Pi^{RC}_{Gene}$ and $Pi^{RN}_{Gene}$ is very small, i.e., there is no much difference in the gene between these two groups, $Lod_{Gene}$ is 0. It can be seen that $Lod_{Gene}$ obviously deviates from 0, and then the gene can be preliminarily considered to be an important gene that causes the differentiation of these two groups.

As described above, the statistics was performed on the $Lod_{Gene}$ values of the over 30,000 genes in HG18, respectively, then were sorted in descending order, and top-ranked genes were obtained by screening.

$F_{ST}$ (Fixation index) is mainly used to evaluate inter-group genomic distances and differences among populations, is an index for measuring the degree of inter-population differentiation, and is developed from a special case of the application of F-test by Sewall Wright in 1922.

The null hypothesis of $F_{ST}$ is that when a group is not differentiated, the difference between the frequencies of the intra-group and the inter-group second allele base of a polymorphic site is not significant. There are many methods for calculating $F_{ST}$, and though the specific calculation methods are different the basic theories are consistent i.e. the definition given by Hudson 1922:

$$F_{ST} = \frac{\Pi_{\text{Between}} - \Pi_{\text{Within}}}{\Pi_{\text{Between}}}$$

In that, $\Pi_{\text{Between}}$ represents that one sample is extracted from two groups, respectively, to form one pair, the difference of SNP genotypes of this pair of samples is calculated, so the differences of SNP genotypes of all paired samples can be calculated, and finally, the average, i.e., $\Pi_{\text{Between}}$, is evaluated.

$\Pi_{\text{Within}}$ represents that 2 samples are extracted from one group, respectively, to form one pair, the difference of SNP genotypes of this pair of samples is calculated, so the differences of SNP genotypes of all paired samples can be calculated, and finally, the average, i.e., $\Pi_{\text{Within}}$, is evaluated.

If there are two groups, $\Pi_{\text{Within}}$ is calculated at first for the two groups, respectively, and then accumulated.

In conjunction with the data structure of the currently existing SNP set, based on the above-mentioned principle, the derived formula is as follows:

$$F_{ST} = \frac{\Pi_{\text{Between}} - \Pi_{\text{Within}}}{\Pi_{\text{Between}}}$$

$$= 1 - \frac{\Pi_{\text{Within}}}{\Pi_{\text{Between}}} = 1 - \frac{\left[ \sum_j \binom{n_j}{2} \sum_i 2 \frac{n_{ij}}{n_{ij}-1} x_{ij}(1-x_{ij}) \right] \bigg/ \sum_j \binom{n_j}{2}}{\sum_i 2 \frac{n_i}{n_i-1} x_i(1-x_i)}$$

In the above formula, $x_{ij}$ is the frequency of the minor allele of SNP Site *i* in Group *j*; $n_{ij}$ is the physical position of SNP Site *i* on chromosomes in Group *j*; and $n_j$ is the sum number of SNP sites for analysis in Group *j*.

In that, the variable *j* had RC and RN, and an SNP position judged finally was substituted for the variable *i*. The $Fst_{Gene}$ value of each gene was calculated with a gene as a unit, and then the $Fst_{Gene}$ values of the over 30,000 genes in HG18 were sorted and top-ranked genes were obtained by screening.

In the case of missing data, the evaluation on the frequencies of SNP sites was not precise, thus rendering that $F_{ST}$ could not reflect sensitively the original attributes of data. According to a method adopted in a reference document (Sequencing of 50 Human Exomes Reveals Adaptation to High Altitude. Science, 2 July 2010, 329, 75-78), the log of $F_{ST}$ was taken, and a third group (the present embodiment introduced part of data in the 1000 Genomes, and the data of genomes of persons from Beijing were recorded as B) was introduced to define *Pbs,* the formula being as follows:

$$T = -\log(1 - Fst)$$

That is, *Fst* of the three groups by comparison between any two was as follows:

$$T_{RC-RN} = -\log(1 - Fst_{RC-RN})$$

$$T_{RC-B} = -\log(1 - Fst_{RC-B})$$

$$T_{RB-B} = -\log(1 - Fst_{RN-B})$$

At this time, the formula of *Pbs* was as follows:

$$Pbs = \frac{T_{RC-RN} + T_{RC-B} - T_{RN-B}}{2}$$

With a gene as a calculation unit, statistics was performed on the $Pbs_{Gene}$ values of the over 30,000 genes in HG18, respectively, and then sorting is performed and top-ranked genes were obtained by screening.

1-12. Gene function analysis

According to at least one of the above three test values, the embodiment obtained important genes by screening according to *Lod, Fst* and *Pbs,* and functional analyses were performed, respectively. It was judged whether these genes are affected in certain pathways, and then associated with the pathogenesis of renal cancer.

**Embodiment 2: Single leukemia cell classification and screening**

2-1. Location of reads

Exome sequencing of a 30× depth was performed on each single cancer cell, the obtained result of reads was aligned to the sequence of a reference genome (the human genome HG18) using the SOAPaligner 2.0 alignment software. Because human SNPs account for two thousandths and the read length of Reads was about 100 bp, during SOAP alignment, we set that each piece of Reads had at most 2 mismatches and Gap was not allowed to occur, so as to ensure the accuracy of the alignment of Reads with the reference genome.

2-2. Basic data statistics

A total of 53 cancer cells and 8 oral epithelial cells (normal cells) were sequenced. Table 5 is the numerical value information of the exome sequencing coverage and depth of each cell sample.

Table 5 The exome sequencing coverage and depth of each cell sample

| Cell sample | Coverage | Depth | Cell sample | Coverage | Depth |
|---|---|---|---|---|---|
| ET-56 | 0.95 | 43.00 | ET-90 | 0.80 | 27.00 |
| ET-52 | 0.94 | 41.00 | ET-1 | 0.79 | 14.00 |
| ET-60 | 0.94 | 39.00 | ET-24 | 0.79 | 18.00 |
| ET-74 | 0.94 | 34.00 | ET-30 | 0.79 | 18.00 |
| ET-66 | 0.93 | 33.00 | ET-89 | 0.78 | 30.00 |
| ET-69 | 0.92 | 30.00 | ET-18 | 0.76 | 17.00 |
| ET-100 | 0.91 | 40.00 | ET-8 | 0.75 | 16.00 |
| ET-61 | 0.90 | 37.00 | ET-78 | 0.74 | 29.00 |

(continued)

| Cell sample | Coverage | Depth | Cell sample | Coverage | Depth |
|---|---|---|---|---|---|
| ET-63 | 0.90 | 33.00 | ET-37 | 0.74 | 21.16 |
| ET-70 | 0.90 | 26.00 | ET-20 | 0.73 | 18.00 |
| ET-93 | 0.90 | 38.00 | ET-88 | 0.73 | 27.00 |
| ET-97 | 0.90 | 35.00 | ET-9 | 0.73 | 19.00 |
| ET-86 | 0.89 | 40.00 | ET-26 | 0.72 | 22.00 |
| ET-45 | 0.88 | 35.00 | ET-31 | 0.70 | 19.00 |
| ET-50 | 0.88 | 39.00 | ET-72 | 0.69 | 25.00 |
| ET-80 | 0.88 | 42.00 | ET-19 | 0.68 | 17.00 |
| ET-44 | 0.86 | 37.00 | ET-73 | 0.67 | 12.00 |
| ET-54 | 0.86 | 42.00 | ET-36 | 0.66 | 19.00 |
| ET-82 | 0.86 | 41.00 | ET-35 | 0.64 | 18.00 |
| ET-22 | 0.85 | 24.00 | ET-21 | 0.63 | 16.00 |
| ET-6 | 0.85 | 17.00 | ET-27 | 0.62 | 17.00 |
| ET-87 | 0.85 | 34.00 | ET-15 | 0.60 | 16.00 |
| ET-16 | 0.84 | 23.00 | NC-30 | 0.46 | 22.00 |
| ET-4 | 0.84 | 15.00 | NC-7 | 0.32 | 6.01 |
| ET-43 | 0.84 | 25.00 | NC-17 | 0.29 | 15.00 |
| ET-5 | 0.84 | 17.00 | NC-29 | 0.25 | 8.62 |
| ET-25 | 0.83 | 20.00 | NC-5 | 0.24 | 4.65 |
| ET-94 | 0.83 | 40.00 | NC-28 | 0.21 | 4.06 |
| ET-3 | 0.81 | 23.00 | NC-14 | 0.21 | 5.40 |
| ET-91 | 0.81 | 31.00 | NC-8 | 0.21 | 5.85 |
| ET-29 | 0.80 | 18.00 | | | |

2-3. Data filtering

The same as that in Embodiment 1

2-4. Judgment on individual genotype

The same as that in Embodiment 1

2-5. SNP dataset

When an SNP dataset was being determined, considering that the number of leukemia cells was relatively large, the coverage rate of exons of the genome of each single cell was not very high, and SNPs were determined based on each individual, we selected relatively strict criteria to screen the obtained data.
The criteria were as follows:
in the Soapsnp software, the quality value of the consistent genotype of each site was not less than 20, and the p value for the rank test was not less than 1%; and for SNPs of heterozygous variation: the genotype of a site is different from the reference genome, and the major allele's sequencing quality value was not less than 20, and the sequencing depth was not less than 6, the minor allele's sequencing quality value was not less than 20, the sequencing depth was not less than 2, and the ratio of sequencing depths of two genotypes was within a range of 0.2-5.
The greater the quality value was, the more accurate the genotyping was, and generally, when same was greater than 20, the error rate was below one ten-thousandth and could be ignored.

After reliable SNPs were obtained by screening using the above criteria, according to the position information in the SNP dataset of the reference genome, sites were determined, and genotyping data of each site of each cell was extracted to generate a genotype file. The format of the file is shown as Table 3.

2-6. Group structure analysis

According to the genotype file on the SNPs of cell group mutation, we classified the various cells using a plurality of commonly used methods in group analysis by bioinformatics, respectively.

2-6.1. Tree construction by the neighbor-joining (N-J) method

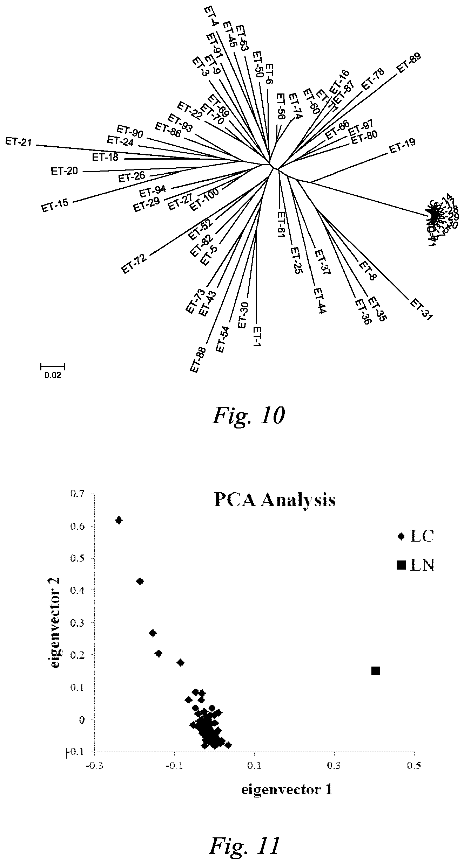

A schematic view of classification relationships of 53 cancer cells and 8 normal cells in the present invention is shown as Figure 10, in which ET-T1 represents a cancer tissue and NC-T1 represents a normal tissue.

2-6.2. Principal components analysis (PCA)

A clustering schematic view of cancer cells and normal cells in the present invention is shown as Figure 11, in which LC represents a cancer cell and LN represents a normal cell.

According to the above group analysis results, the information of cell samples was extracted, and contentious cells were excluded (unclearly classified or evident outlier samples). It was shown from the above group structures that the sampling was normal and the classification was reasonable.

2-6.3. Subgroup classification

According to the shape or condition of the phylogenetic tree, all the 53 cancer cells could be clearly divided into 4 classes of subgroups, indicating that there existed real differences in cancer cells. Different cell subgroups in one and the same cancer tissue could be obtained by classification using the single cell analysis method.

2-7. Selection of high-confidence somatic cell mutation

High-confidence somatic cell mutation sites were screened from the genotype file, and the criteria were as follows:

the normal cells having a consistent homozygous genotype, there existing two or more heterozygous mutations or homozygous mutations in the cancer cells, and a third homozygous genotype and a heterozygous genotype inconsistent with the two homozygous genotypes being not allowed to occur. For instance, for the normal cells, the genotype is A, or the mutation type is A -> C, and then, only three genotypes can occur in the cancer cells, i.e., A, C and M, and the number of C and M is not less than 2. We refer to such sites as high-confidence somatic mutation (HCSM). It was the exome sequencing technology that we used, and thus, sites that were not in exon regions were filtered to obtain a total of 2,296 HCSMs, in which there were 879 synonymous sites and 1,417 non-synonymous sites (containing missense mutation and truncation mutation sites), and the non-synonymous/synonymous mutation ratio was 1.61, shown as Table 6.

Table 6 High-confidence somatic cell mutations

| | |
|---|---|
| High-confidence somatic cell mutations (coding region) | 2296 |
| synonymous mutations | 879 |
| missense mutations | 1354 |
| truncation mutations | 63 |
| non-synonymous/synonymous mutation ratio | 1.612059 |

2-8. Gene function and pathway analysis

That was a downstream analysis which could be performed after cell classification and screening, with the position information of gene mutation sites and the number of non-synonymous mutation sites existing in each gene as the criteria for gene function enrichment, the Webgestalt on-line analytical tool was used to study gene functions and pathways affected by the mutations (bioinfo.vanderbilt.edu/webgestalt/option.php), and it was found that the mutations were mainly concentrated in genes with the following 8 classes of functions.

Table 7 The result of gene function analysis of mutation sites

| GO classification | GO Number | Functional annotation | Significance value |
|---|---|---|---|
| biological process | GO:0006996 | organelle organization | 0.0026 |
| biological process | GO:0016043 | cellular component organization | 0.0026 |
| molecular function | GO:0005198 | structural molecule activity | 0.0021 |
| cellular component | GO:0044430 | cytoskeletal part | 0.0003 |
| cellular component | GO:0043228 | non-membrane-bounded organelle | 0.0008 |
| cellular component | GO:0043232 | intracellular non-membrane-bounded organelle | 0.0008 |
| cellular component | GO:0005856 | cytoskeleton | 0.0008 |
| cellular component | GO:0044420 | extracellular matrix part | 0.0044 |

It was found by pathway analysis that the mutant genes were mainly concentrated in 10 pathways, the vast majority of which were related to the pathogenesis of cancers:

Metabolic pathways

ECM-receptor interaction

Pathways in cancer

Viral myocarditis

Type I diabetes mellitus

MAPK signaling pathway

Focal adhesion

Pantothenate and CoA biosynthesis

Cell adhesion molecules (CAMs)

Allograft rejection

2-9. Prediction of gene functions of mutation sites

We selected non-synonymous mutation sites in exon regions, and used the SIFT (sift.jcvi.org/) software to predict the functions of genes corresponding to these mutation sites. The result was divided into 4 cases, shown as Table 8 below:

Table 8 The result of prediction of gene functions of mutation sites

| Gene name | Position on chromosomes | Functional prediction of mutations |
|---|---|---|
| SLC2A7 | chr1,9000880 | DAMAGING (damage to gene functions present) |
| PLEKHN1 | chr1,899101 | DAMAGING *Warning!Low confidence (low confidence damage) |
| PANK4 | chr1,2436894 | N/A (unable to be judged) |
| ACOT7 | chr1,6322197 | TOLERATED (little influence of the variation on gene functions) |

The 4 cases were: damage to gene functions present, low confidence damage, little influence of the variation on gene functions and unable to be judged. We selected mutation sites with damage to functions and verified the genes occurring in the above-mentioned function enrichment and pathways by subsequent experiments.

**Industrial Applicability**

The technical solutions of the present invention can be effectively applied in cell classification and screening of genes associated with mutagenesis.

In the description of the present specification, the reference terms "one embodiment", "some embodiments", "exemplary embodiment", "example", "particular example" or "some examples" and other expressions mean particular features, structures, materials or characteristics described in conjunction with the embodiment or example are contained in at least one embodiment or example of the present invention. In the present specification, the schematic representations of the above-mentioned terms are not necessary to mean the same embodiment or example. Moreover, the particular features, structures, materials or characteristics described can be combined in any one or more embodiments or examples in an appropriate manner.

**Claims**

1. A method of classifying single cells according to genotype comprising the steps of:

sequencing the whole genomes of a plurality of single cell samples from the same group, respectively, so as to obtain reads from each single cell sample;
aligning the reads from each single cell sample to the sequence of a reference genome, respectively, and performing data filtering on said reads so as to determine a consistent genotype of each single cell, wherein the criteria of said data filtering are:

when a plurality of pairs of duplicated paired-end reads are present, and the sequences of the plurality of pairs of reads are fully consistent, randomly selecting one pair of reads, and removing the other duplicated paired-end reads from said plurality of pairs of reads; and/or removing reads which are not uniquely aligned onto the sequence of said reference genome; and further, on the basis of the consistent genotype of each single cell, identifying SNPs and storing the result in a SNP dataset; and
using the SNP dataset to determine the genotype of each cell at a site corresponding to the position of a SNP in the SNP dataset of the reference genome; and further
selecting a SNP site associated with cell mutation, wherein selecting a SNP site associated with cell mutation involves removing at least one of the following items from the SNP dataset of said group: non inter-group SNP sites whereby the base types of all individuals are identical and all are SNPs relative to a reference sequence, sites of loss of heterozygosity, and published SNP sites; and
on the basis of the genotypes of said single cells at the site associated with cell mutation, performing group structure analysis;
wherein determining a consistent genotype of each single cell includes:

on the basis of said filtered reads, determining a possibility of a genotype of each single cell sample in a target region;
on the basis of possibilities of genotypes of all the single cell samples in the target region, determining a pseudo-genome containing each site of all the cell samples; and
selecting a genotype with a maximum probability from said pseudo-genome as the consistent genotype of each single cell sample.

2. A method of classifying single cells as claimed in claims 1 , wherein on the basis of the SNP dataset of said group, determining a corresponding genotype for each cell at a site corresponding to a position in an SNP dataset of the reference genome, further comprises screening of said SNP dataset according to the following criteria:

the quality value of the consistent genotype of each site being not less than 20, and the p value for the rank test being not less than 1%; and
for SNPs of heterozygous variation: the major allele's sequencing quality value being not less than 20, and the sequencing depth being not less than 6, the minor allele's sequencing quality value being not less than 20, the sequencing depth being not less than 2, and the ratio of sequencing depths of two genotypes being within a range of 0.2-5; optionally further comprising the following step after distinguishing cells of extracting the information of each cell sample, and excluding contentious cells.

**3.** A device for classifying single cells by genotype, comprising:

a) a data filtering module, said data filtering module being suitable for aligning reads from each single cell sample to the sequence of a reference genome, respectively, and performing data filtering on said reads, in which the reads of said each single cell sample are obtained by sequencing the whole genomes of a plurality of single cell samples, respectively, wherein the criteria of said data filtering are:

when a plurality of pairs of duplicated paired-end reads are present, and the sequences of the plurality of pairs of reads are fully consistent, randomly selecting one pair of reads, and removing the other duplicated paired-end reads in said plurality of pairs of reads; and/or removing reads which are not uniquely aligned onto the sequence of said reference genome;

b) a genotype determination module, said genotype determination module being suitable for determining a consistent genotype of each single cell sample on the basis of the filtered reads; and
on the basis of the consistent genotype of each single cell, identifying SNPs and storing the result in a SNP dataset; and suitable for determining the consistent genotype of said each single cell through the following items:

on the basis of said filtered reads, determining a possibility of a genotype of each single cell sample in a target region;
on the basis of possibilities of genotypes of all the single cell samples in the target region, determining a pseudo-genome containing each site of all the samples; and
selecting a genotype with a maximum probability from said pseudo-genome as the consistent genotype of each single cell sample;

c) a genotype file extraction module, said genotype file extraction module being suitable for determining the genotype of each cell at a site corresponding to the position of a SNP in the SNP dataset of the reference genome; and further
d) a classification module, said classification module being suitable for classifying said single cells on the basis of a pre-selected SNP site associated with cell mutation, and on the basis of the genotypes of said single cells at the site, wherein selecting a SNP site associated with cell mutation involves removing at least one of the following items from the SNP dataset of said group:

non inter-group SNP sites whereby the base types of all individuals are identical and all are SNPs relative to a reference sequence, sites of loss of heterozygosity, and published SNP sites.

**4.** A device as claimed in claim 3 , wherein said genotype file extraction module is suitable for screening said SNP dataset according to the following criteria:

the quality value of the consistent genotype of each site being not less than 20, and the p value for the rank test being not less than 1%; and
for SNPs of heterozygous variation: the major allele's sequencing quality value being not less than 20, and the sequencing depth being not less than 6, the minor allele's sequencing quality value being not less than 20, the sequencing depth being not less than 2, and the ratio of sequencing depths of two genotypes being within a range of 0.2-5.

**5.** A device as claimed in claim 3 or claim 4, wherein said classification module is further suitable for extracting the information of each cell sample, and excluding contentious cells.

**Patentansprüche**

**1.** Verfahren zum Klassifizieren einzelner Zellen nach deren Genotyp, wobei das Verfahren die folgenden Schritte umfasst:

Sequenzieren der vollständigen Genome einer Mehrzahl von Proben einzelner Zellen aus der gleichen Gruppe, um aus jeder Probe einer einzelnen Zelle Reads zu erhalten;

Ausrichten der Reads jeder Probe einer einzelnen Zelle mit der Sequenz eines entsprechenden Referenzgenoms, und Durchführen einer Datenfilterung an den Reads, um einen übereinstimmenden Genotyp jeder einzelnen Zelle zu bestimmen, wobei die Datenfilterungskriterien folgende sind:

wenn mehrere Paare duplizierter Reads mit gepaarten Enden vorhanden sind und die Sequenzen der mehreren Paare von Reads vollständig übereinstimmen, wahlfreies Auswählen eines Paares von Reads und Entfernen der anderen duplizierten Reads mit gepaarten Enden aus den mehreren Paaren von Reads; und/oder Entfernen von Reads, die nicht eindeutig auf die Sequenz des Referenzgenoms ausgerichtet sind; und ferner auf der Basis des übereinstimmenden Genotyps jeder einzelnen Zelle, Identifizieren von SNPs und Speichern des Ergebnisses in der SNP-Datenmenge; und

Verwenden der SNP-Datenmenge zur Bestimmung des Genotyps jeder Zelle an einer Stelle, die der Position eines SNP in der SNP-Datenmenge des Referenzgenoms entspricht; und ferner

Auswählen einer SNP-Stelle, die einer Zellmutation zugeordnet ist, wobei das Auswählen einer SNP-Stelle, die einer Zellmutation zugeordnet ist, das Entfernen wenigstens eines der folgenden Elemente aus der SNP-Datenmenge der Gruppe beinhaltet: Nicht-Intergruppen-SNP-Stellen, wobei die Grundtypen aller Individuen identisch sind und alle SNPs im Verhältnis zu einer Referenzsequenz sind, Stellen mit Verlust der Heterozygotie und veröffentlichte SNP-Stellen; und

auf der Basis der Genotypen der einzelnen Zellen an der Zelle, die Zellmutation zugeordnet ist, Ausführen einer Gruppenstrukturanalyse;

wobei das Bestimmen eines übereinstimmenden Genotyps jeder einzelnen Zelle folgendes aufweist:

auf der Basis der gefilterten Reads, Bestimmen einer Wahrscheinlichkeit für einen Genotyp jeder Probe einer einzelnen Zelle in einem Zielbereich;

auf der Basis der Wahrscheinlichkeiten von Genotypen aller Proben einzelner Zellen in dem Zielbereich, Bestimmen eines Pseudogenoms, das jede Stelle aller Zellproben enthält; und

Auswählen eines Genotyps mit einer maximalen Wahrscheinlichkeit aus dem Pseudogenom als den übereinstimmenden Genotyp jeder Probe einer einzelnen Zelle.

2. Verfahren zum Klassifizieren einzelner Zellen nach Anspruch 1, wobei auf der Basis der SNP-Datenmenge der Gruppe das Bestimmen eines entsprechenden Genotyps für jede Zelle an einer Stelle, die einer Position in einer SNP-Datenmenge des Referenzgenoms entspricht, ferner das Screening der SNP-Datenmenge gemäß den folgenden Kriterien umfasst:

der Qualitätswert des übereinstimmenden Genotyps jeder Stelle ist nicht kleiner als 20, und der p-Wert für den Rangordnungstest ist nicht kleiner als 1%; und

für SNPs mit heterozygoten Variation: der Sequenzierungsqualitätswert der dominanten Allele ist nicht kleiner als 20, und die Sequenzierungstiefe ist nicht kleiner als 6, der Sequenzierungsqualitätswert der rezessiven Allele ist nicht kleiner als 20, die Sequenzierungstiefe ist nicht kleiner als 2, und das Verhältnis der Sequenzierungstiefen von zwei Genotypen liegt in einem Bereich von 0,2 bis 5; ferner optional den folgenden Schritt umfassend nach dem Unterscheiden der Zellen des Extrahierens der Informationen jeder Zellenprobe und des Ausschließens kontroverser Zellen.

3. Vorrichtung zum Klassifizieren einzelner Zellen nach deren Genotyp, wobei die Vorrichtung folgendes umfasst:

a) ein Datenfiltermodul, wobei das Datenfiltermodul geeignet ist zum Ausrichten der Reads jeder Probe einer einzelnen Zelle mit der Sequenz eines entsprechenden Referenzgenoms, und Durchführen einer Datenfilterung an den Reads, wobei die Reads jeder Probe einer einzelnen Zelle erhalten werden durch entsprechende Sequenzierung der vollständigen Genome mehrerer Proben einzelner Zellen, wobei die Datenfilterungskriterien folgende sind:

wenn mehrere Paare duplizierter Reads mit gepaarten Enden vorhanden sind und die Sequenzen der mehreren Paare von Reads vollständig übereinstimmen, wahlfreies Auswählen eines Paares von Reads und Entfernen der anderen duplizierten Reads mit gepaarten Enden aus den mehreren Paaren von Reads; und/oder Entfernen von Reads, die nicht eindeutig auf die Sequenz des Referenzgenoms ausgerichtet sind;

b) ein Genotypbestimmungsmodul, wobei das Genotypbestimmungsmodul einen übereinstimmenden Genotyp jeder Probe einer einzelnen Zelle auf der Basis der gefilterten Reads bestimmen kann; und

auf der Basis des übereinstimmenden Genotyps jeder einzelnen Zelle, Identifizieren von SNPs und Speichern des Ergebnisses in der SNP-Datenmenge; und geeignet ist zur Bestimmung des übereinstimmenden Genotyps jeder einzelnen Zelle durch die folgenden Elemente:

auf der Basis der gefilterten Reads, Bestimmen einer Wahrscheinlichkeit für einen Genotyp jeder Probe einer einzelnen Zelle in einem Zielbereich;
auf der Basis der Wahrscheinlichkeiten von Genotypen aller Proben einzelner Zellen in dem Zielbereich, Bestimmen eines Pseudogenoms, das jede Stelle aller Zellproben enthält; und
Auswählen eines Genotyps mit einer maximalen Wahrscheinlichkeit aus dem Pseudogenom als den übereinstimmenden Genotyp jeder Probe einer einzelnen Zelle;

c) ein Genotyp-Dateiextraktionsmodul, wobei das GenotypDateiextraktionsmodul geeignet ist zum Bestimmen des Genotyps jeder Zelle an einer Stelle, die der Position eines SNP in der SNP-Datenmenge des Referenzgenoms entspricht; und ferner
d) ein Klassifizierungsmodul, wobei das Klassifizierungsmodul geeignet ist zum Klassifizieren der einzelnen Zellen auf der Basis einer vorab ausgewählten SNP-Stelle, die der Zellmutation zugeordnet ist, und auf der Basis der Genotypen der einzelnen Zelle an der Stelle, wobei das Auswählen einer ausgewählten SNP-Stelle, die der Zellmutation zugeordnet ist, das Entfernen wenigstens eines der folgenden Elemente aus der SNP-Datenmenge der Gruppe beinhaltet:

Nicht-Intergruppen-SNP-Stellen, wobei die Grundtypen aller Individuen identisch sind und alle SNPs im Verhältnis zu einer Referenzsequenz sind, Stellen mit Verlust der Heterozygotie und veröffentlichte SNP-Stellen.

4. Vorrichtung nach Anspruch3, wobei das Genotypdateiextraktionsmodul geeignet ist für ein Screening der SNP-Datenmenge gemäß den folgenden Kriterien:

der Qualitätswert des übereinstimmenden Genotyps jeder Stelle ist nicht kleiner als 20, und der p-Wert für den Rangordnungstest ist nicht kleiner als 1%; und
für SNPs mit heterozygoten Variation: der Sequenzierungsqualitätswert der dominanten Allele ist nicht kleiner als 20, und die Sequenzierungstiefe ist nicht kleiner als 6, der Sequenzierungsqualitätswert der rezessiven Allele ist nicht kleiner als 20, die Sequenzierungstiefe ist nicht kleiner als 2, und das Verhältnis der Sequenzierungstiefen von zwei Genotypen liegt in einem Bereich von 0,2 bis 5.

5. Vorrichtung nach Anspruch 3 oder Anspruch 4, wobei das Klassifizierungsmodul ferner geeignet ist zum Extrahieren der Informationen jeder Zellenprobe und zum Ausschließen kontroverser Zellen.

## Revendications

1. Procédé de classification de cellules individuelles conformément à leur génotype comprenant les étapes de :

séquençage des génomes complets d'une pluralité d'échantillons de cellules individuelles du même groupe, respectivement, de façon à obtenir des lectures de chaque échantillon de cellules individuelles ;
alignement des lectures provenant de chaque échantillon de cellules individuelles avec la séquence d'un génome de référence, respectivement, et la réalisation d'un filtrage de données desdites lectures de façon à déterminer un génotype cohérent de chaque cellule individuelle, dans lequel les critères dudit filtrage de données sont :

lorsqu'une pluralité de doubles paires de lectures à extrémité appariée sont présentes, et que les séquences de la pluralité de paires de lectures sont entièrement cohérentes, sélection aléatoire d'une paire de lectures, et suppression des autres doubles de lectures à extrémité appariée à partir de ladite pluralité de la paire de lecture ; et/ou suppression des lectures qui ne sont pas individuellement alignées avec la séquence dudit génome de référence ; et en outre, sur la base du génotype cohérent de chaque cellule individuelle, identification de SNP et stockage du résultat dans un ensemble de données SNP ; et
utilisation de l'ensemble de données SNP afin de déterminer le génotype de chaque cellule au niveau d'un site correspondant à la position d'un SNP dans l'ensemble de données SNP du génome de référence ; et en outre
sélection d'un site SNP associé à une mutation cellulaire, dans lequel la sélection d'un site SNP associé à une mutation cellulaire implique la suppression d'au moins un des articles suivants de l'ensemble de données SNP dudit groupe ; des sites SNP non intergroupés moyennant quoi les types de base de tous les individus sont identiques et tous sont des SNP par rapport à une séquence de référence, des sites de perte d'hétérozygotie, et des sites SNP publiés ; et

sur la base des génotypes desdites cellules individuelles au niveau du site associé à la mutation cellulaire, réalisation d'une analyse de structure de groupe ;

dans lequel la détermination d'un génotype cohérent de chaque cellule individuelle comprend :

sur la base desdites lectures filtrées, détermination d'une possibilité d'un génotype de chaque échantillon de cellules individuelles dans une région cible ;

sur la base des possibilités de génotypes de tous les échantillons de cellules individuelles dans la région cible, détermination d'un pseudo-génome contenant chaque site de tous les échantillons de cellules ;et

sélection d'un génotype ayant une probabilité maximum à partir dudit pseudo-génome comme le génotype cohérent de chaque échantillon de cellules individuelles.

2. Procédé de classification de cellules individuelles selon la revendication 1, dans lequel sur la base de l'ensemble de données SNP dudit groupe, la détermination d'un génotype correspondant pour chaque cellule au niveau d'un site correspondant à une position dans un ensemble de données SNP du génome de référence, comprend en outre le criblage dudit ensemble de données SNP conformément aux critères suivants :

la valeur de qualité du génotype cohérent de chaque site étant supérieure ou égale à 20, et la valeur p pour le test de rang étant supérieure ou égale à 1 % ; et

pour des SNP présentant une variation hétérozygote : la valeur importante de qualité de séquençage d'allèle étant supérieure ou égale à 20, et la profondeur de séquençage étant supérieure ou égale à 6, la valeur mineure de qualité de séquençage d'allèle étant supérieure ou égale à 20, la profondeur de séquençage étant supérieure ou égale à 2, et le rapport des profondeurs de séquençage de deux génotypes étant compris dans une plage allant de 0,2 à 5 ; comprenant en outre éventuellement l'étape suivante après la distinction de cellules d'extraction des informations de chaque échantillon de cellules, et l'exclusion de cellules litigieuses.

3. Dispositif permettant de classifier des cellules individuelles par génotype, comprenant :

a) un module de filtrage de données, ledit module de filtrage de données permettant l'alignement de lectures provenant de chaque échantillon de cellules individuelles avec la séquence d'un génome de référence, respectivement, et la réalisation du filtrage de données sur lesdites lectures, dans lequel les lectures de chacun desdits échantillons de cellules individuelles sont obtenues par le séquençage des génomes complets d'une pluralité d'échantillons de cellules individuelles, respectivement, dans lequel les critères dudit filtrage de données sont :

lorsqu'une pluralité de doubles paires de lectures à extrémité appariée sont présentes, et que les séquences de la pluralité de paires de lectures sont entièrement cohérentes, sélection aléatoire d'une paire de lectures, et suppression des autres doubles de lectures à extrémité appariée dans ladite pluralité de paires de lectures ; et/ou la suppression de lectures qui ne sont pas individuellement alignées avec la séquence dudit génome de référence ;

b) un module de détermination de génotype, ledit module de détermination de génotype permettant de déterminer un génotype cohérent de chaque échantillon de cellules individuelles sur la base des lectures filtrées ; et sur la base du génotype cohérent de chaque cellule individuelle, identification des SNP et stockage du résultat dans un ensemble de données SNP ; et permettant de déterminer le génotype cohérent de chacune desdites cellules individuelles grâce aux articles suivants :

sur la base desdites lectures filtrées, détermination d'une possibilité d'un génotype de chaque échantillon de cellules individuelles dans une région cible ;

sur la base de possibilités de génotypes de tous les échantillons de cellules individuelles dans la région cible, détermination d'un pseudo-génome contenant chaque site de tous les échantillons ; et

sélection d'un génotype ayant une probabilité maximum à partir dudit pseudo-génome comme le génotype cohérent de chaque échantillon de cellules individuelles ;

c) un module d'extraction de fichiers de génotype, ledit module d'extraction de fichiers de génotype permettant de déterminer le génotype de chaque cellule au niveau d'un site correspondant à la position d'un SNP dans l'ensemble de données SNP du génome de référence ; et en outre

d) un module de classification, ledit module de classification permettant de classifier lesdites cellules individuelles sur la base d'un site SNP présélectionné associé à une mutation cellulaire, et sur la base des génotypes desdites cellules individuels au niveau du site, dans lequel la sélection d'un site SNP associé à une mutation cellulaire implique la suppression d'au moins un des articles suivants à partir de l'ensemble de données SNP dudit groupe :

des sites SNP non intergroupés moyennant quoi les types de base de tous les individus sont identiques et tous sont des SNP par rapport à une séquence de référence, des sites de perte d'hétérozygotie, et des sites SNP publiés.

4. Dispositif selon la revendication 3, dans lequel ledit module d'extraction de fichiers de génotype est apte au criblage dudit ensemble de données SNP conformément aux critères suivants :

la valeur de qualité du génotype cohérent de chaque site étant supérieure ou égale à 20, et la valeur p du test de rang étant supérieure ou égale à 1 % ;

pour des SNP présentant une variation hétérozygote : la valeur importante de qualité de séquençage d'allèle étant supérieure ou égale à 20, et la profondeur de séquençage étant supérieure ou égale à 6, la valeur mineure de qualité de séquençage d'allèle étant supérieure ou égale à 20, la profondeur de séquençage étant supérieure ou égale à 2, et le rapport des profondeurs de séquençage de deux génotypes étant compris dans une plage allant de 0,2 à 5.

5. Dispositif selon la revendication 3 ou la revendication 4, dans lequel ledit module de classification permet en outre d'extraire des informations de chaque échantillon de cellules, et d'exclure des cellules litigieuses.

Reference genome

Fig. 1

Reference genome

Reads D

Reads E

Fig. 2

*Fig. 3*

*Fig. 4*

*Fig. 5*

*Fig. 6*

*Fig. 7*

*Fig. 8*

*Fig. 9*

Fig. 10

Fig. 11

| Data filtering module | Classification module | Screening module |
|---|---|---|

| Genotype determination module | Genotype file extraction module |
|---|---|

*Fig. 12*

| Acquisition unit |
|---|

| Computing unit | Sorting unit |
|---|---|

*Fig. 13*

**EP 2 749 655 B2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *A tutorial on Principal Components Analysis,* February 2002

- **J. SAMBROOK.** Molecular Cloning: A Laboratory Manual. Science Press
- *Science,* 02 July 2010, vol. 329, 75-78